(19)

Europäisches
Patentamt
European
Patent Office
Office européen
des brevets

(11) **EP 4 231 303 A1**

(12) # EUROPEAN PATENT APPLICATION

(43) Date of publication:
**23.08.2023 Bulletin 2023/34**

(21) Application number: **22158024.4**

(22) Date of filing: **22.02.2022**

(51) International Patent Classification (IPC):
**G16C 20/10** (2019.01)  **G16C 20/30** (2019.01)

(52) Cooperative Patent Classification (CPC):
**G16C 20/10; G16C 20/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Borealis AG**
**1020 Vienna (AT)**

(72) Inventors:
• **TOULOUPIDIS, Vasileios**
  **4021 Linz (AT)**

• **KAUFMANN, Christoph**
  **84489 Burghausen (DE)**
• **CARLSSON, Niclas**
  **444 86 Stenungsund (SE)**
• **SKALEN, Staffan**
  **444 86 Stenungsund (SE)**

(74) Representative: **Maiwald GmbH**
**Elisenhof**
**Elisenstraße 3**
**80335 München (DE)**

(54) **METHOD AND APPARATUS FOR POLYMERIZATION PROCESS MONITORING AND MODEL PREDICTIVE CONTROL**

(57)  The present invention is related to a method for polymerization process monitoring and model predictive control, the method comprising the steps of: providing (S1) at least one set of parameters comprising catalyst information, kinetic modelling information, and calculated polymer property information; and modeling (S2) a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types.

Fig. 1

Fig. 2

EP 4 231 303 A1

**Description**

FIELD OF THE INVENTION

The present invention relates to polymerization process monitoring and model predictive control and in particular to method for polymerization process monitoring and model predictive control.

BACKGROUND OF THE INVENTION

Control methods for the polymerization reactors are known, comprising of a mathematical model for the polymerization process as well as an optimizer for defining the best manipulative option.

The detailed kinetics of the polymer reaction engineering model are for instance described in the following documents:

The article by Touloupidis V., published 2014, titled "Catalytic Olefin Polymerization Process Modeling: Multi-Scale Approach and Modeling Guidelines for Micro-Scale/Kinetic Modeling", published in Macromol. React. Eng., 8, pp. 508-527, describes multi-scale polymerization process modeling framework, comprising of polymerization kinetics (micro-scale), system thermodynamics (meso-scale), and reactor performance (macro-scale) as well as to present the methodology for developing a mathematical model for catalytic olefin polymerization.

The article by Soares J.B.P., published 2014, titled "The Use of Instantaneous Distributions in Polymerization Reaction Engineering", published in Macromol. React. Eng., 8, 235-259, describes how the method of instantaneous distributions can be used to model the microstructures of polymers made under different polymerization conditions.

The article by Soares J., Touloupidis V., published 2019, titled "Polymerization Kinetics and the Effect of Reactor Residence Time on Polymer Microstructure", published in "Jeremic D., Prades F. and Albunia A. ed. Multimodal Polymers with Supported Catalysts: Design and Production", Springer International Publishing, describes the polymerization kinetics and the effect of reactor residence time on polymer microstructure.

The article by Touloupidis V., Rittenschober G., Paulik C., published 2020, titled "An Integrated PRE Methodology for Capturing the Reaction Performance of Single- and Multi-site Type Catalysts Using Bench-Scale Polymerization Experiments", Macromol. React. Eng., describes systems and methods for capturing the reaction performance of single- and multi-site type catalysts.

The model is able to predict polymerization rate and polymer product molecular properties (molecular weight distribution, comonomer content distribution and average comonomer content) based on first principles (e.g., mass balance equations) as well as a number of secondary polymer properties, including polymer density and melt index, based on and a series of correlations.

SUMMARY OF THE INVENTION

The foregoing and other objects are solved by the subject-matter of the present invention as defined by the independent claims. Further embodiments are defined by the dependent claims. The present invention therefore allows enabling a new quality control concept aiming in better defining the polymer properties, under the scope of both for process monitoring and control.

According to a first aspect of the present invention, a method is provided, a method for polymerization process monitoring and model predictive control.

The method comprises the steps of: providing at least one set of parameters comprising catalyst information, kinetic modelling information, and calculated polymer property information and modelling a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types.

In other words, the present invention advantageously provides as differences compared to prior art, enabling a new quality control concept aiming in better defining the polymer properties, under the scope of both for process monitoring and polymer property oriented model predictive control.

According to the concept of the present invention, the kinetic parameters that are able to describe the reaction performance of the catalyst are fed into the model together with the operating conditions. The model is able to predict the expected reactive concentration values in the reactor(s), the polymerization rate per reactor (and consequently the split) as well as the polymer microstructure per reactor, for instance as molecular weight distribution, MWD, comonomer content, CC.

Furthermore, a rheology model provides a second level prediction for the expected complex viscosity at the exit of each reactor (for a range of shear rates). This model prediction is compared at regular intervals (typically every 4-6 hours) with the experimental value of the fast frequency sweep measurement of the corresponding reactor sample as well as with the corresponding desired fast frequency sweep set-point, enabling the control of the selected manipulated variable.

According to one exemplary embodiment of the present invention, the method further comprises the step of predicting, based on the modelled reaction performance, an expected reactive concentration value, a polymerization rate per reactor, or a polymer microstructure per reactor.

According to one exemplary embodiment of the present invention, the step of modelling of the reaction performance of the catalyst further comprises the step of using a rheology model for predicting an expected complex viscosity.

According to one exemplary embodiment of the present invention, the set of parameters further comprises a feed rate used for the polymerization process, a temperature used for the polymerization process, or a pressure used for the polymerization process.

According to one exemplary embodiment of the present invention, the method further comprises the step of modelling at least one value of a molecular property based on the modelled reaction performance.

According to one exemplary embodiment of the present invention, the method further comprises the step of modelling at least one value of a rheological property based on the modelled reaction performance.

According to one exemplary embodiment of the present invention, the modelled reaction performance is used for process monitoring by means of an optimizer, preferably the optimizer is connected to a controller for controlling the polymerization process.

According to one exemplary embodiment of the present invention, the modelled reaction performance is used for process monitoring by means of predicting process and polymer parameters. In addition, an optimizer is connected to a controller for controlling the polymerization process.

According to one exemplary embodiment of the present invention, the modelled reaction performance is used in an offline mode.

According to a second aspect of the present invention, a computer program product is provided comprising computer-readable instructions which, when loaded and executed on processor, performs the method according to any one of the embodiments of the first aspect or the first aspect as such.

According to a third aspect of the present invention, an apparatus is provided, the apparatus configured for polymerization process monitoring and model predictive control, the apparatus comprising a data memory and a processor.

The data memory is configured to provide at least one set of parameters comprising catalyst information, kinetic modelling information, and calculated polymer property information.

The processor is configured to model a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types.

According to one embodiment of the present invention, the processor is further configured to predict, based on the modelled reaction performance, an expected reactive concentration value, a polymerization rate per reactor, or a polymer microstructure per reactor.

According to one embodiment of the present invention, the processor is further configured to model the reaction performance of the catalyst in terms of using a rheology model for predicting an expected complex viscosity.

According to one embodiment of the present invention, the set of parameters further comprises a feed rate used for the polymerization process, a temperature used for the polymerization process, or a pressure used for the polymerization process.

According to one embodiment of the present invention, the processor is further configured to model at least one value of a molecular property based on the modelled reaction performance. According to one embodiment of the present invention, the processor is further configured to model at least one value of a rheological property based on the modelled reaction performance.

A computer program performing the method of the present invention may be stored on a computer-readable medium. A computer-readable medium may be a floppy disk, a hard disk, a CD, a DVD, an USB (Universal Serial Bus) storage device, a RAM (Random Access Memory), a ROM (Read Only Memory) and an EPROM (Erasable Programmable Read Only Memory).

A computer-readable medium may also be a data communication network, for example the Internet, which allows downloading a program code, with a connection via WLAN or 3G/4G or any other wireless data technology.

The methods, systems and devices described herein may be implemented as software in a Digital Signal Processor, DSP, in a micro-controller or in any other side-processor or as hardware circuit within an application specific integrated circuit, ASIC, CPLD or FPGA.

The present invention can be implemented in digital electronic circuitry, or in computer hardware, firmware, software, or in combinations thereof, e.g. in available hardware of conventional mobile devices or in new hardware dedicated for processing the methods described herein.

BRIEF DESCRIPTION OF THE DRAWINGS

A more complete appreciation of the invention and the attendant advantages thereof will be more clearly understood by reference to the following schematic drawings, which are not to scale, wherein:

Fig. 1 shows a schematic diagram of a method for polymerization process monitoring and model predictive control;
Fig. 2 shows a schematic diagram of an apparatus for polymerization process monitoring and model predictive control according to an exemplary embodiment of the invention;
Fig. 3 shows a schematic diagram of a model predictive control concept according to an exemplary embodiment of the invention; and
Fig. 4 shows a schematic diagram of a detailed kinetics model predictive control concept according to an exemplary embodiment of the invention;

DETAILED DESCRIPTION OF EMBODIMENTS

The illustration in the drawings is schematically and not to scale. In different drawings, similar or identical elements are provided with the same reference numerals.

Generally, identical parts, units, entities or steps are provided with the same reference symbols in the figures.

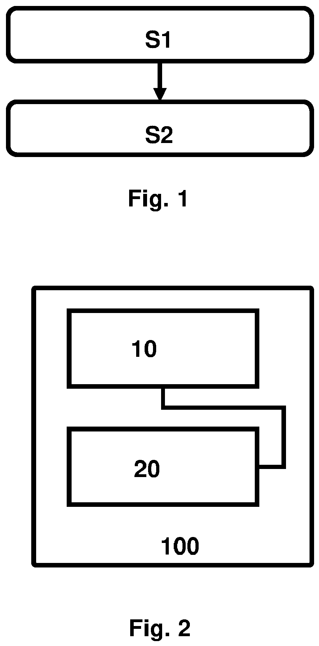

Fig. 1 shows a schematic diagram of a method for polymerization process monitoring and model predictive control.

In particular, Fig. 1 shows a method for polymerization process monitoring and model predictive control, the method comprising the following steps.

As a first step of the method, providing S1 at least one set of parameters comprising catalyst information, kinetic modelling information, and calculated polymer property information is conducted.

As a second step of the method, modeling S2 a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types is performed.

Fig. 2 shows a schematic diagram of an apparatus for polymerization process monitoring and model predictive control.

An apparatus 100 for polymerization process monitoring and model predictive control is provided, the apparatus 100 comprises a data memory 10, which is configured to provide at least one set of parameters comprising catalyst information, kinetic modelling information, and calculated polymer property information.

Further, the apparatus 100 comprises a processor 20, which is configured to model a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types.

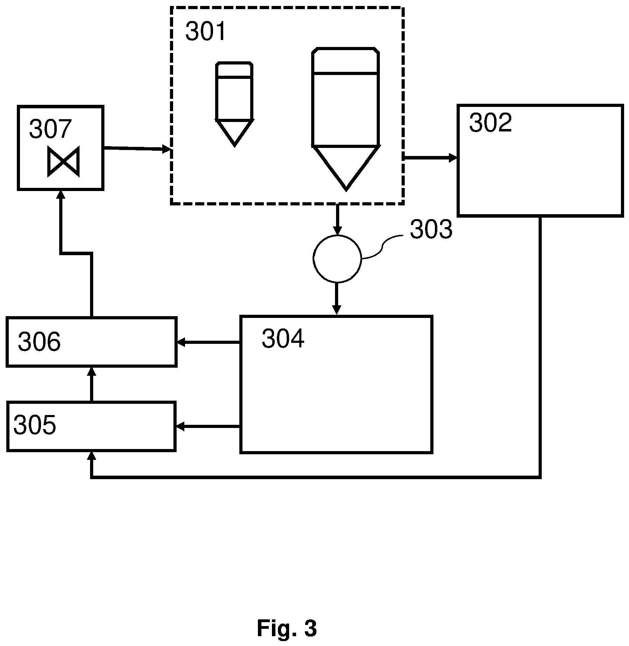

Fig. 3 shows a schematic diagram of a model predictive control concept according to an exemplary embodiment of the invention.

The kinetic model contains a whole reaction network that is calculated the modelled process 301, the characterization data 302 contains data on MWD, CCD, and rheology. Controlled variables 303 are measured continuously. Online measurements 304 on temperature, pressure or concentrations are provided to the model 305 configured to provide a model predictive control and the controller 306 configured to provide process control.

The specific model 305 is setup with kinetic parameters and feed into the controller 306. Feedback to the process is provided with the manipulative variable 307 to the process reactor.

According to an exemplary embodiment of the invention, in contrast to the currently used models, the new kinetic model contains kinetic descriptions for a reaction network of fundamental reactions taking place at the catalysts active centers.

Furthermore it is assumed, that the behavior of a Ziegler-Natta catalyst can be described by a discrete number of different site types, each of them acting as 'single site catalyst'.

The kinetics for the whole reaction network is calculated for each site type individually. The macroscopic behavior of the catalyst is the sum of the contributions of the different site types.

For the description of the model, the following nomenclature is used:

$C_p^k$     Concentration of potential sites of type k

$C_0^k$     Concentration of activated sites of type k

$C_{act,i}^{k}$     Concentration of reactive sites of type k, last monomer inserted to polymer chain is of type i

$C_{D}^{k}$     Concentration of deactivated sites of type k

A     Concentration of co-catalyst

$M_1$     Monomer 1 concentration (Ethylene)

$M_2$     Monomer 2 concentration

$M_3$     Monomer 3 concentration

$P_1, P_2, P_3$     Polymerblock of monomer 1, monomer 2, monomer 3

$H_2$     Concentration of hydrogen

The reaction network consists of the following elementary reactions:

*Site Activation:*

By co-catalyst:

$$C_{p}^{k} + A \xrightarrow{k_{aA}^{k}} C_{0}^{k} + B \tag{1}$$

By Monomer:

$$C_{p}^{k} + M_1 \xrightarrow{k_{a1}^{k}} C_{act,1}^{k} + P_1 \tag{2}$$

$$C_{p}^{k} + M_2 \xrightarrow{k_{a2}^{k}} C_{act,2}^{k} + P_2 \tag{3}$$

$$C_{p}^{k} + M_3 \xrightarrow{k_{a3}^{k}} C_{act,3}^{k} + P_3 \tag{4}$$

*Chain Initiation:*

$$C_{0}^{k} + M_1 \xrightarrow{k_{0,1}^{k}} C_{act,1}^{k} + P_1 \tag{5}$$

$$C_{0}^{k} + M_2 \xrightarrow{k_{0,2}^{k}} C_{act,2}^{k} + P_2 \tag{6}$$

$$C_{0}^{k} + M_3 \xrightarrow{k_{0,3}^{k}} C_{act,3}^{k} + P_3 \tag{7}$$

*Chain Propagation:*

$$C_{act,1}^{k} + M_1 \xrightarrow{k_{p11}^{k}} C_{act,1}^{k} + P_1 \tag{8}$$

$$C_{act,1}^{k} + M_2 \xrightarrow{k_{p12}^{k}} C_{act,2}^{k} + P_2 \tag{9}$$

$$C_{act,1}^{k} + M_3 \xrightarrow{k_{p13}^{k}} C_{act,3}^{k} + P_3 \tag{10}$$

$$C_{act,2}^{k} + M_1 \xrightarrow{k_{p21}^{k}} C_{act,1}^{k} + P_1 \tag{11}$$

$$C_{act,2}^{k} + M_2 \xrightarrow{k_{p22}^{k}} C_{act,2}^{k} + P_2 \tag{12}$$

$$C_{act,2}^{k} + M_3 \xrightarrow{k_{p23}^{k}} C_{act,3}^{k} + P_3 \tag{13}$$

$$C_{act,3}^{k} + M_1 \xrightarrow{k_{p31}^{k}} C_{act,1}^{k} + P_1 \tag{14}$$

$$C_{act,3}^{k} + M_2 \xrightarrow{k_{p32}^{k}} C_{act,2}^{k} + P_2 \tag{15}$$

$$C_{act,3}^{k} + M_3 \xrightarrow{k_{p33}^{k}} C_{act,3}^{k} + P_3 \tag{16}$$

_Chain Transfer:_
By hydrogen:

$$C_{act,1}^{k} + H_2 \xrightarrow{k_{trH1}^{k}} C_0^{k} \tag{17}$$

$$C_{act,2}^{k} + H_2 \xrightarrow{k_{trH2}^{k}} C_0^{k} \tag{18}$$

$$C_{act,3}^{k} + H_2 \xrightarrow{k_{trH3}^{k}} C_0^{k} \tag{19}$$

Spontaneous:

$$C_{act,1}^{k} \xrightarrow{k_{trsp,1}^{k}} C_0^{k} \tag{20}$$

$$C_{act,2}^{k} \xrightarrow{k_{trsp,2}^{k}} C_0^{k} \tag{21}$$

$$C_{act,3}^{k} \xrightarrow{k_{trsp,3}^{k}} C_0^{k} \tag{22}$$

_Deactivation:_

By hydrogen:

$$C_{act,1}^k + H_2 \xrightarrow{\;k_{DH1}^k\;} C_D^k \tag{23}$$

$$C_{act,2}^k + H_2 \xrightarrow{\;k_{DH2}^k\;} C_D^k \tag{24}$$

$$C_{act,3}^k + H_2 \xrightarrow{\;k_{DH3}^k\;} C_D^k \tag{25}$$

$$C_0^k + H_2 \xrightarrow{\;k_{DH0}^k\;} C_D^k \tag{26}$$

Spontaneous:

$$C_{act,1}^k \xrightarrow{\;k_{Dsp,1}^k\;} C_D^k \tag{27}$$

$$C_{act,2}^k \xrightarrow{\;k_{Dsp,2}^k\;} C_D^k \tag{28}$$

$$C_{act,3}^k \xrightarrow{\;k_{Dsp,3}^k\;} C_D^k \tag{29}$$

$$C_0^k \xrightarrow{\;k_{Dsp,0}^k\;} C_D^k \tag{30}$$

All of the above reactions are assumed to be of first order. Having calculated the rates for each of the reactions at each of the site types the following values are accessible, describing the overall conversion of reactants.

Total amount of polymer consisting of monomer 1 formed per time interval:

$$\frac{dP_1}{dt} = \sum_{k=1}^{6} k_{0,1}^k \cdot e^{\frac{-Ea_{0,1}^k}{R \cdot T}} \cdot C_0^k \cdot M_1 + \sum_{k=1}^{6} k_{p11}^k \cdot e^{\frac{-Ea_{p11}^k}{R \cdot T}} \cdot C_{act,1}^k \cdot M_1$$

$$+ \sum_{k=1}^{6} k_{p21}^k \cdot e^{\frac{-Ea_{p21}^k}{R \cdot T}} \cdot C_{act,2}^k \cdot M_1 + \sum_{k=1}^{6} k_{p31}^k \cdot e^{\frac{-Ea_{p31}^k}{R \cdot T}} \cdot C_{act,3}^k \cdot M_1 \tag{31}$$

$$+ \sum_{k=1}^{6} k_{a1}^k \cdot e^{\frac{-Ea_{a1}^k}{R \cdot T}} \cdot C_P^k \cdot M_1$$

Total amount of polymer consisting of monomer 2 formed per time interval:

$$\frac{dP_2}{dt} = \sum_{k=1}^{6} k_{0,2}^k \cdot e^{\frac{-Ea_{0,2}^k}{R \cdot T}} \cdot C_0^k \cdot M_2 + \sum_{k=1}^{6} k_{p12}^k \cdot e^{\frac{-Ea_{p12}^k}{R \cdot T}} \cdot C_{act,1}^k \cdot M_2$$

$$+ \sum_{k=1}^{6} k_{p22}^k \cdot e^{\frac{-Ea_{p22}^k}{R \cdot T}} \cdot C_{act,2}^k \cdot M_2 + \sum_{k=1}^{6} k_{p32}^k \cdot e^{\frac{-Ea_{p32}^k}{R \cdot T}} \cdot C_{act,3}^k \cdot M_2 \qquad (32)$$

$$+ \sum_{k=1}^{6} k_{a2}^k \cdot e^{\frac{-Ea_{a2}^k}{R \cdot T}} \cdot C_P^k \cdot M_2$$

Total amount of polymer consisting of monomer 3 formed per time interval:

$$\frac{dP_3}{dt} = \sum_{k=1}^{6} k_{0,3}^k \cdot e^{\frac{-Ea_{0,3}^k}{R \cdot T}} \cdot C_0^k \cdot M_3 + \sum_{k=1}^{6} k_{p13}^k \cdot e^{\frac{-Ea_{p13}^k}{R \cdot T}} \cdot C_{act,1}^k \cdot M_3$$

$$+ \sum_{k=1}^{6} k_{a3}^k \cdot e^{\frac{-Ea_{a3}^k}{R \cdot T}} \cdot C_P^k \cdot M_3 \qquad (33)$$

The amount of cocatalyst reacted per time interval:

$$\frac{dA}{dt} = \sum_{k=1}^{6} k_{aA}^k \cdot e^{\frac{-Ea_{aA}^k}{R \cdot T}} \cdot C_p^k \cdot A \qquad (34)$$

The amount of hydrogen reacted per time interval:

$$\frac{dH_2}{dt} = - \sum_{k=1}^{6} k_{trH1}^k \cdot e^{\frac{-Ea_{trH1}^k}{R \cdot T}} \cdot C_{act,1}^k \cdot H_2 - \sum_{k=1}^{6} k_{trH2}^k \cdot e^{\frac{-Ea_{trH2}^k}{R \cdot T}} \cdot C_{act,2}^k \cdot H_2$$

$$- \sum_{k=1}^{6} k_{trH3}^k \cdot e^{\frac{-Ea_{trH3}^k}{R \cdot T}} \cdot C_{act,3}^k \cdot H_2 - \sum_{k=1}^{6} k_{DH1}^k \cdot e^{\frac{-Ea_{DH1}^k}{R \cdot T}} \cdot C_{act,1}^k \cdot H_2 \qquad (35)$$

$$- \sum_{k=1}^{6} k_{DH2}^k \cdot e^{\frac{-Ea_{DH2}^k}{R \cdot T}} \cdot C_{act,2}^k \cdot H_2 - \sum_{k=1}^{6} k_{DH3}^k \cdot e^{\frac{-Ea_{DH3}^k}{R \cdot T}} \cdot C_{act,3}^k \cdot H_2$$

$$- \sum_{k=1}^{6} k_{DH0}^k \cdot e^{\frac{-Ea_{DH0}^k}{R \cdot T}} \cdot C_0^k \cdot H_2$$

Per site type the following expressions can be derived:
The change of potential sites of type k per time interval:

$$\frac{dC_p^k}{dt} = -k_{aA}^k \cdot e^{\frac{-Ea_{aA}^k}{R \cdot T}} \cdot C_p^k \cdot A - k_{a1}^k \cdot e^{\frac{-Ea_{a1}^k}{R \cdot T}} \cdot C_p^k \cdot M_1 \tag{36}$$

$$-k_{a2}^k \cdot e^{\frac{-Ea_{a2}^k}{R \cdot T}} \cdot C_p^k \cdot M_2 - k_{a3}^k \cdot e^{\frac{-Ea_{a3}^k}{R \cdot T}} \cdot C_p^k \cdot M_3$$

The change of activated sites of type k per time interval:

$$\frac{dC_0^k}{dt} = k_{aA}^k \cdot e^{\frac{-Ea_{aA}^k}{R \cdot T}} \cdot C_p^k \cdot A - k_{0,1}^k \cdot e^{\frac{-Ea_{0,1}^k}{R \cdot T}} \cdot C_0^k \cdot M_1 - k_{0,2}^k \cdot e^{\frac{-Ea_{0,2}^k}{R \cdot T}} \cdot C_0^k \cdot M_2$$

$$- k_{0,3}^k \cdot e^{\frac{-Ea_{0,3}^k}{R \cdot T}} \cdot C_0^k \cdot M_3 + k_{trH1}^k \cdot e^{\frac{-Ea_{trH1}^k}{R \cdot T}} \cdot C_{act,1}^k \cdot H_2 + k_{trH2}^k \cdot e^{\frac{-Ea_{trH2}^k}{R \cdot T}} \cdot C_{act,2}^k \cdot H_2 \tag{37}$$

$$+ k_{trH3}^k \cdot e^{\frac{-Ea_{trH3}^k}{R \cdot T}} \cdot C_{act,3}^k \cdot H_2 + k_{trsp,1}^k \cdot e^{\frac{-Ea_{trsp,1}^k}{R \cdot T}} \cdot C_{act,1}^k + k_{trsp,2}^k \cdot e^{\frac{-Ea_{trsp,2}^k}{R \cdot T}} \cdot C_{act,2}^k$$

$$+ k_{trsp,3}^k \cdot e^{\frac{-Ea_{trsp,3}^k}{R \cdot T}} \cdot C_{act,3}^k - k_{DH0}^k \cdot e^{\frac{-Ea_{DH0}^k}{R \cdot T}} \cdot C_0^k \cdot H_2 - k_{Dsp,0}^k \cdot e^{\frac{-Ea_{Dsp,0}^k}{R \cdot T}} \cdot C_0^k$$

The change of deactivated sites of type k per time interval:

$$\frac{dC_D^k}{dt} = k_{DH1}^k \cdot e^{\frac{-Ea_{DH1}^k}{R \cdot T}} \cdot C_{act,1}^k \cdot H_2 + k_{DH2}^k \cdot e^{\frac{-Ea_{DH2}^k}{R \cdot T}} \cdot C_{act,2}^k \cdot H_2$$

$$+ k_{DH3}^k \cdot e^{\frac{-Ea_{DH3}^k}{R \cdot T}} \cdot C_{act,3}^k \cdot H_2 + k_{Dsp,1}^k \cdot e^{\frac{-Ea_{Dsp,1}^k}{R \cdot T}} \cdot C_{act,1}^k + k_{Dsp,2}^k \cdot e^{\frac{-Ea_{Dsp,2}^k}{R \cdot T}} \cdot C_{act,2}^k \tag{38}$$

$$+ k_{Dsp,3}^k \cdot e^{\frac{-Ea_{Dsp,3}^k}{R \cdot T}} \cdot C_{act,3}^k + k_{DH0}^k \cdot e^{\frac{-Ea_{DH0}^k}{R \cdot T}} \cdot C_0^k \cdot H_2 + k_{Dsp,0}^k \cdot e^{\frac{-Ea_{Dsp,0}^k}{R \cdot T}} \cdot C_0^k$$

The change of reactive sites of type k per time interval:

$$\frac{dC_{act,1}^k}{dt} = k_{0,1}^k \cdot e^{\frac{-Ea_{0,1}^k}{R \cdot T}} \cdot C_0^k \cdot M_1 - k_{p12}^k \cdot e^{\frac{-Ea_{p12}^k}{R \cdot T}} \cdot C_{act,1}^k \cdot M_2$$

$$+ k_{p21}^k \cdot e^{\frac{-Ea_{p21}^k}{R \cdot T}} \cdot C_{act,2}^k \cdot M_1 - k_{p13}^k \cdot e^{\frac{-Ea_{p13}^k}{R \cdot T}} \cdot C_{act,1}^k \cdot M_3 + k_{p31}^k \cdot e^{\frac{-Ea_{p31}^k}{R \cdot T}} \cdot C_{act,3}^k \cdot M_1 \tag{39}$$

$$- k_{trH1}^k \cdot e^{\frac{-Ea_{trH1}^k}{R \cdot T}} \cdot C_{act,1}^k \cdot H_2 - k_{trsp1}^k \cdot e^{\frac{-Ea_{trsp1}^k}{R \cdot T}} \cdot C_{act,1}^k - k_{DH1}^k \cdot e^{\frac{-Ea_{DH1}^k}{R \cdot T}} \cdot C_{act,1}^k \cdot H_2$$

$$- k_{Dsp,1}^k \cdot e^{\frac{-Ea_{Dsp,1}^k}{R \cdot T}} \cdot C_{act,1}^k + k_{a1}^k \cdot e^{\frac{-Ea_{a1}^k}{R \cdot T}} \cdot C_P^k \cdot M_1$$

The change of reactive sites of type k (last inserted monomer = monomer 2) per time interval:

$$\frac{dC_{act,2}^k}{dt} = k_{0,2}^k \cdot e^{\frac{-Ea_{0,2}^k}{R \cdot T}} \cdot C_0^k \cdot M_2 + k_{p12}^k \cdot e^{\frac{-Ea_{p12}^k}{R \cdot T}} \cdot C_{act,1}^k \cdot M_2$$

$$- k_{p21}^k \cdot e^{\frac{-Ea_{p21}^k}{R \cdot T}} \cdot C_{act,2}^k \cdot M_1 - k_{p23}^k \cdot e^{\frac{-Ea_{p23}^k}{R \cdot T}} \cdot C_{act,2}^k \cdot M_3 + k_{p32}^k \cdot e^{\frac{-Ea_{p32}^k}{R \cdot T}} \cdot C_{act,3}^k \cdot M_2 \qquad (40)$$

$$- k_{trH2}^k \cdot e^{\frac{-Ea_{trH2}^k}{R \cdot T}} \cdot C_{act,2}^k \cdot H_2 - k_{trsp2}^k \cdot e^{\frac{-Ea_{trsp2}^k}{R \cdot T}} \cdot C_{act,2}^k - k_{DH2}^k \cdot e^{\frac{-Ea_{DH2}^k}{R \cdot T}} \cdot C_{act,2}^k \cdot H_2$$

$$- k_{Dsp,2}^k \cdot e^{\frac{-Ea_{Dsp,2}^k}{R \cdot T}} \cdot C_{act,2}^k + k_{a2}^k \cdot e^{\frac{-Ea_{a2}^k}{R \cdot T}} \cdot C_P^k \cdot M_2$$

The change of reactive sites of type k (last inserted monomer = monomer 3) per time interval:

$$\frac{dC_{act,3}^k}{dt} = k_{0,3}^k \cdot e^{\frac{-Ea_{0,3}^k}{R \cdot T}} \cdot C_0^k \cdot M_3 + k_{p13}^k \cdot e^{\frac{-Ea_{p13}^k}{R \cdot T}} \cdot C_{act,1}^k \cdot M_3$$

$$- k_{p31}^k \cdot e^{\frac{-Ea_{p31}^k}{R \cdot T}} \cdot C_{act,3}^k \cdot M_1 - k_{p32}^k \cdot e^{\frac{-Ea_{p32}^k}{R \cdot T}} \cdot C_{act,3}^k \cdot M_2 + k_{p23}^k \cdot e^{\frac{-Ea_{p23}^k}{R \cdot T}} \cdot C_{act,2}^k \cdot M_3 \qquad (41)$$

$$- k_{trH3}^k \cdot e^{\frac{-Ea_{trH3}^k}{R \cdot T}} \cdot C_{act,3}^k \cdot H_2 - k_{trsp3}^k \cdot e^{\frac{-Ea_{trsp3}^k}{R \cdot T}} \cdot C_{act,3}^k - k_{DH3}^k \cdot e^{\frac{-Ea_{DH3}^k}{R \cdot T}} \cdot C_{act,3}^k \cdot H_2$$

$$- k_{Dsp,3}^k \cdot e^{\frac{-Ea_{Dsp,3}^k}{R \cdot T}} \cdot C_{act,3}^k + k_{a3}^k \cdot e^{\frac{-Ea_{a3}^k}{R \cdot T}} \cdot C_P^k \cdot M_3$$

The amount of polymer consisting of monomer 1 formed at site type k per time interval:

$$\frac{dP_{1,k}}{dt} = k_{0,1}^k \cdot e^{\frac{-Ea_{0,1}^k}{R \cdot T}} \cdot C_0^k \cdot M_1 + k_{p11}^k \cdot e^{\frac{-Ea_{p11}^k}{R \cdot T}} \cdot C_{act,1}^k \cdot M_1 \qquad (42)$$

$$+ k_{p21}^k \cdot e^{\frac{-Ea_{p21}^k}{R \cdot T}} \cdot C_{act,2}^k \cdot M_1 + k_{p31}^k \cdot e^{\frac{-Ea_{p31}^k}{R \cdot T}} \cdot C_{act,3}^k \cdot M_1 + k_{a1}^k \cdot e^{\frac{-Ea_{a1}^k}{R \cdot T}} \cdot C_P^k \cdot M_1$$

The amount of polymer consisting of monomer 2 formed at site type k per time interval:

$$\frac{dP_{2,k}}{dt} = k_{0,2}^k \cdot e^{\frac{-Ea_{0,2}^k}{R \cdot T}} \cdot C_0^k \cdot M_2 + k_{p12}^k \cdot e^{\frac{-Ea_{p12}^k}{R \cdot T}} \cdot C_{act,1}^k \cdot M_2 \qquad (43)$$

$$+ k_{p22}^k \cdot e^{\frac{-Ea_{p22}^k}{R \cdot T}} \cdot C_{act,2}^k \cdot M_2 + k_{p32}^k \cdot e^{\frac{-Ea_{p32}^k}{R \cdot T}} \cdot C_{act,3}^k \cdot M_2 + k_{a2}^k \cdot e^{\frac{-Ea_{a2}^k}{R \cdot T}} \cdot C_P^k \cdot M_2$$

The amount of polymer consisting of monomer 3 formed at site type k per time interval:

$$\frac{dP_{3,k}}{dt} = k_{0,3}^{k} \cdot e^{\frac{-Ea_{0,3}^{k}}{R \cdot T}} \cdot C_{0}^{k} \cdot M_{3} + k_{p13}^{k} \cdot e^{\frac{-Ea_{p13}^{k}}{R \cdot T}} \cdot C_{act,1}^{k} \cdot M_{3} \qquad (44)$$

$$+ k_{p23}^{k} \cdot e^{\frac{-Ea_{p23}^{k}}{R \cdot T}} \cdot C_{act,2}^{k} \cdot M_{3} + k_{p33}^{k} \cdot e^{\frac{-Ea_{p33}^{k}}{R \cdot T}} \cdot C_{act,3}^{k} \cdot M_{3} + k_{a3}^{k} \cdot e^{\frac{-Ea_{a3}^{k}}{R \cdot T}} \cdot C_{P}^{k} \cdot M_{3}$$

The sum of propagation rates at site type k:

$$R_{\text{Pr}op,k} = \frac{dP_{1,k}}{dt} + \frac{dP_{2,k}}{dt} + \frac{dP_{3,k}}{dt} \qquad (45)$$

The sum of chain transfer (and deactivation) rates at site type k:

$$R_{Trans,k} = k_{trH1}^{k} \cdot e^{\frac{-Ea_{trH1}^{k}}{R \cdot T}} \cdot C_{act,1}^{k} \cdot H_{2} + k_{trH2}^{k} \cdot e^{\frac{-Ea_{trH2}^{k}}{R \cdot T}} \cdot C_{act,2}^{k} \cdot H_{2}$$

$$+ k_{trH3}^{k} \cdot e^{\frac{-Ea_{trH3}^{k}}{R \cdot T}} \cdot C_{act,3}^{k} \cdot H_{2} + k_{trsp,1}^{k} \cdot e^{\frac{-Ea_{trsp,1}^{k}}{R \cdot T}} \cdot C_{act,1}^{k} + k_{trsp,2}^{k} \cdot e^{\frac{-Ea_{trsp,2}^{k}}{R \cdot T}} \cdot C_{act,2}^{k}$$

$$+ k_{trsp,3}^{k} \cdot e^{\frac{-Ea_{trsp,3}^{k}}{R \cdot T}} \cdot C_{act,3}^{k} + k_{DH1}^{k} \cdot e^{\frac{-Ea_{DH1}^{k}}{R \cdot T}} \cdot C_{act,1}^{k} \cdot H_{2} + k_{DH2}^{k} \cdot e^{\frac{-Ea_{DH2}^{k}}{R \cdot T}} \cdot C_{act,2}^{k} \cdot H_{2} \qquad (46)$$

$$+ k_{DH3}^{k} \cdot e^{\frac{-Ea_{DH3}^{k}}{R \cdot T}} \cdot C_{act,3}^{k} \cdot H_{2} + k_{Dsp,1}^{k} \cdot e^{\frac{-Ea_{Dsp,1}^{k}}{R \cdot T}} \cdot C_{act,1}^{k} + k_{Dsp,2}^{k} \cdot e^{\frac{-Ea_{Dsp,2}^{k}}{R \cdot T}} \cdot C_{act,2}^{k}$$

$$+ k_{Dsp,3}^{k} \cdot e^{\frac{-Ea_{Dsp,3}^{k}}{R \cdot T}} \cdot C_{act,3}^{k}$$

The instantaneous comonomer content of monomer 2 at site type k:

$$CC_{2,k} = \frac{\dfrac{P_{2,k}}{dt}}{\left( \dfrac{P_{1,k}}{dt} + \dfrac{P_{2,k}}{dt} + \dfrac{P_{3,k}}{dt} \right)} \qquad (47)$$

The instantaneous comonomer content of monomer 3 at site type k:

$$CC_{3,k} = \frac{\dfrac{P_{3,k}}{dt}}{\left( \dfrac{P_{1,k}}{dt} + \dfrac{P_{2,k}}{dt} + \dfrac{P_{3,k}}{dt} \right)} \qquad (48)$$

Having calculated the instantaneous comonomer contents at site type k, the instantaneous average molecular weight of the polymer formed at site type k can be calculated:

$$AMW_{K} = M_{1} \cdot \left( 1 - CC_{2,k} - CC_{3,k} \right) + M_{2} \cdot CC_{2,k} + M_{3} \cdot CC_{3,k} \qquad (49)$$

With $M_{1}$, $M_{2}$ and $M_{3}$ being the molecular weights of monomer 1, monomer 2 and monomer 3.

From the instantaneous average molecular weight the instantaneous number average molecular weight ($M_{n,k}$) can be derived, using $R_{\mathrm{Prop},k}$ and $R_{Trans,k}$.

$$M_{n,k} = AMW_k \cdot \frac{R_{\mathrm{Prop},k}}{R_{Trans,k}} \tag{50}$$

Taking into account that the polydispersity index for the single site types is 2 the instantaneous weight average molecular weight for site type k ($M_{w,k}$) is:

$$M_{w,k} = 2 \cdot M_{n,k} \tag{51}$$

The instantaneous molecular weight distribution for site type k is given by the following Schulz-Flory distribution:

$$MWD_k(i) = \frac{R_{\mathrm{Prop},k} \cdot AMW_k}{\frac{dP_1}{dt} + \frac{dP_2}{dt} + \frac{dP_3}{dt}} \cdot 2.3026 \cdot i^2 \cdot \left(\frac{R_{Trans,k}}{R_{\mathrm{Prop},k}}\right)^2 \cdot 10^{\frac{-i \cdot R_{Trans,k}}{R_{\mathrm{Prop},k}}} \tag{52}$$

Where i is the chain length of polymer (the X-axis in the distribution).

Taking into account that the polydispersity index for the single site types is 2 the instantaneous weight average molecular weight for site type k ($M_{w,k}$) is:

$$M_{w,k} = 2 \cdot M_{n,k} \tag{51}$$

The instantaneous molecular weight distribution for site type k is given by the following Schulz-Flory distribution:

$$MWD_k(i) = \frac{R_{\mathrm{Prop},k} \cdot AMW_k}{\frac{dP_1}{dt} + \frac{dP_2}{dt} + \frac{dP_3}{dt}} \cdot 2.3026 \cdot i^2 \cdot \left(\frac{R_{Trans,k}}{R_{\mathrm{Prop},k}}\right)^2 \cdot 10^{\frac{-i \cdot R_{Trans,k}}{R_{\mathrm{Prop},k}}} \tag{52}$$

Where i is the chain length of polymer (the X-axis in the distribution).

The total instantaneous MWD of the polymer formed over all site types of the catalyst is the sum of the instantaneous MWDs of the different site types:

$$MWD(i) = \sum_{k=1}^{6} MWD_k(i) \tag{53}$$

The instantaneous comonomer content distribution for monomer 2 over all site types k is given by:

$$CCD2(i) = \sum_{k=1}^{6} \frac{MWD_k(i) \cdot CC_{2,k}}{MWD(i)} \tag{54}$$

And analogously the comonomer content distribution for monomer 3 over all site types k is given by:

$$CCD3(i) = \sum_{k=1}^{6} \frac{MWD_k(i) \cdot CC_{3,k}}{MWD(i)} \tag{55}$$

The rheology method for the prediction of complex viscosity based on the predicted MWD is described in the paper article: Touloupidis V., Wurnitsch C., Albunia A. and Galgali G., 2016, 'Connecting Linear Polymers Molecular Structure to Viscoelastic Properties and Melt Flow Index', Macromol. Theory Simul., 25, pp. 392-402 and in the technical report: Touloupidis V., 2013. BorVisc: Method for predicting polymer rheological properties based on MWD. Case study for LLDPE and HDPE Borealis grades, *2013TR198.*

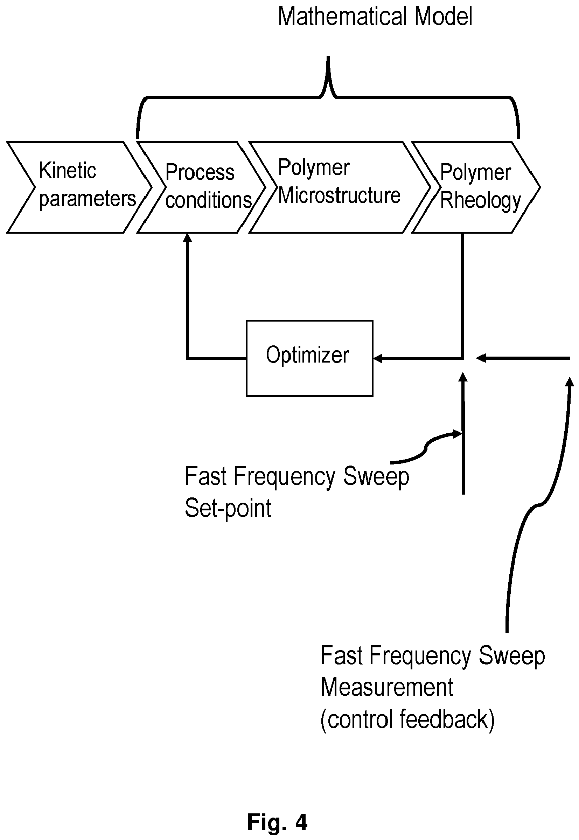

Fig. 4 shows a schematic diagram of a detailed kinetics model predictive control concept according to an exemplary embodiment of the invention.

Monitoring and Control of Polymerization Process

The complexity of the chemical and physical phenomena taking place within a polymerization reactor series, the overlapping effect of the input variables of the system (including flowrates, temperature and pressure) as well as the continuous and dynamic nature of the process require a method for effective monitoring and control (method for manipulating the variables of interest in such a way that the controlled variable(s) value(s) remain within the specified specification range (setpoint) According to the basic feedback control scheme, manipulative actions are based on feeding process output information back to the controller.

This control methodology has been in use over the last 20 years, where feedback information may refer to reactive concentration values, polymer density or melt index value. The control method comprises of a mathematical model for the polymerization process as well as an optimizer for defining the best manipulative option. The model is based on mass balance equations and a series of empirical correlations for the prediction of polymerization rate as well as a number of polymer properties, including polymer density and melt index.

For example, the monomer conversion is a function of temperature, pressure, hydrogen content and comonomer content. The correlation is in the form of a product of a correction factor, a pre-exponential factor, terms that describe the influence of the concentration and/or partial pressures of the monomer and other reaction partners, terms that describe the mass of catalyst inside the reactor and the catalysts activity, and an Arrhenius-type term that captures the influence of temperature. The non-linear behaviour of these parameters is fitted in order to match the observed plant data.

This methodology works well from a control perspective as trend-wise the model is accurate. For example, the current setup offers process conditions / stabilizing control and successful control of polymerization conditions:

- Production rate
- H2/Monomer ratio

However, since detailed kinetic considerations are not included, the model predictions regarding the polymer microstructure and hence its physical and chemical properties such as molecular weight distribution (MWD), comonomer content (CC) and distribution (CCD) and its subsequent rheological behaviour cannot be achieved.

This limitation prevents from reaching property-oriented control capabilities. What is missing is:

- Product property control
- Polymer properties monitoring & control (MWD, CC)
- Rheological properties (complex viscosity, melt index)

The present invention therefore leads to a series of benefits:

    i. Improved control during transitions
    ii. Improved product consistency
    iii. Transitions optimization
    iv. Reducing the need for test runs
    v. Tool for process understanding and trouble-shooting
    vi. Deeper knowledge of the polymer structure
    vii. Increase in product quality performance

The proposed method for polymerization process monitoring and model predictive control offers a new powerful tool for polymerization reactor operation: the proposed method for polymerization process monitoring and model predictive control is able to predict the polymer microstructural characteristics, including MWD, CC across MWD and CCD based on the kinetic parameters of the catalyst in use and the process conditions (feed rates, temperatures, pressure).

Further, the proposed method for polymerization process monitoring and model predictive control is able to predict the complex viscosity (within a range of shear rates) of the produced polymer.

Further, the proposed method for polymerization process monitoring and model predictive control is able to provide predicted values of the molecular and rheological properties that can be further used as the basis for second level structure-property correlations to additional polymer properties (e.g. correlations towards crack resistance, stress at yield, etc.).

Further, the proposed method for polymerization process monitoring and model predictive control can be used for process monitoring providing important information dynamically to the plant operators in the control room.

Further, the proposed method for polymerization process monitoring and model predictive control can be used in an offline mode (not connected to the plant) for engineering studies regarding alternative process conditions or reactor setups (product and process development). Further, the proposed method for polymerization process monitoring and model predictive control can receive control feedback using the fast frequency sweep method closing the loop and enabling model predictive control operation.

Further, the proposed method for polymerization process monitoring and model predictive control enables property-oriented control, targeting to the intrinsic polymer microstructural characteristics (currently not feasible).

Further, the proposed method for polymerization process monitoring and model predictive control is able to increase the model validity of stabilizing control leading to improved control during transitions.

The use of the proposed method provides additional degrees of freedom regarding variables we are able to manipulate since now a method to predict their effect to the process exists (e.g., manipulating split or reactor temperature).

It has to be noted that embodiments of the invention are described with reference to different subject matters. In particular, some embodiments are described with reference to method type claims whereas other embodiments are described with reference to the device type claims.

However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

**Claims**

1. A method for polymerization process monitoring and model predictive control, the method comprising the steps of:

    - providing (S1) at least one set of parameters comprising catalyst information, kinetic modelling information, and calculated polymer property information;
    - modelling (S2) a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types.

2. The method according to claim 1,
   wherein the method further comprises the step of predicting, based on the modelled reaction performance, an expected reactive concentration value, a polymerization rate per reactor, or a polymer microstructure per reactor.

3. The method according to claim 1 or 2,
   wherein the step of modeling (S2) of the reaction performance of the catalyst further comprises the step of using a rheology model for predicting an expected complex viscosity.

4. The method according to any one of the preceding claims,
   wherein the set of parameters further comprises a feed rate used for the polymerization process, a temperature used for the polymerization process, or a pressure used for the polymerization process.

5. The method according to any one of the preceding claims,

   wherein the method further comprises the step of
   modelling (S3) at least one value of a molecular property based on the modelled reaction performance.

6. The method according to any one of the preceding claims,

   wherein the method further comprises the step of
   modelling (S3) at least one value of a rheological property based on the modelled reaction performance.

7. The method according to any one of the preceding claims,
   wherein the modelled reaction performance is used for process monitoring by means of an optimizer, preferably the optimizer is connected to a controller for controlling the polymerization process.

8. The method according to any one of the preceding claims,
   wherein the modelled reaction performance is used in an offline mode.

9. An apparatus (100) for polymerization process monitoring and model predictive control, the apparatus comprising

   - a data memory (10) which is configured to provide at least one set of parameters comprising catalyst information, kinetic modelling information, and calculated polymer property information; and
   - a processor (20), which is configured to model a reaction performance of a catalyst used for a polymerization process using a reaction network, the reaction network comprising multiple reactions of the catalyst, wherein reactions of the catalyst are modelled by a discrete number of multiple site types of the catalyst, wherein the catalyst is modelled as a sum of contributions of the multiple site types.

10. The apparatus according to claim 9,
    wherein the processor (20) is further configured to predict, based on the modelled reaction performance, an expected reactive concentration value, a polymerization rate per reactor, or a polymer microstructure per reactor.

11. The apparatus according to claim 9 or 10,
    wherein the processor (20) is further configured to model the reaction performance of the catalyst in terms of using a rheology model for predicting an expected complex viscosity.

12. The apparatus according to any one of the preceding claims 9 to 11,
    wherein the set of parameters further comprises a feed rate used for the polymerization process, a temperature used for the polymerization process, or a pressure used for the polymerization process.

13. The apparatus according to any one of the preceding claims 9 to 12,
    wherein the processor (20) is further configured to model at least one value of a molecular property based on the modelled reaction performance.

14. The apparatus according to any one of the preceding claims 9 to 13,
    wherein the processor (20) is further configured to model at least one value of a rheological property based on the modelled reaction performance.

15. A computer-readable medium comprising instructions which, when executed by a computer, cause the computer to carry out the steps of the method of any one of the preceding claims 1 to 8.

S1

S2

**Fig. 1**

10

20

100

**Fig. 2**

**Fig. 3**

Mathematical Model

Kinetic parameters

Process conditions

Polymer Microstructure

Polymer Rheology

Optimizer

Fast Frequency Sweep Set-point

Fast Frequency Sweep Measurement (control feedback)

**Fig. 4**

Europäisches
Patentamt

European
Patent Office

Office européen
des brevets

## EUROPEAN SEARCH REPORT

**Application Number**

EP 22 15 8024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | TOULOUPIDIS V. ET AL: "An Integrated PRE Methodology for Capturing the Reaction Performance of Single- and Multi-site Type Catalysts Using Bench-Scale Polymerization Experiments", MACROMOLECULAR REACTION ENGINEERING, vol. 14, no. 6, 31 August 2020 (2020-08-31), page 2000028, XP055961188, ISSN: 1862-832X, DOI: 10.1002/mren.202000028 Retrieved from the Internet: URL:https://onlinelibrary.wiley.com/doi/full-xml/10.1002/mren.202000028> * the whole document * ----- | 1-15 | INV. G16C20/10 G16C20/30 |
| A | RAMANATHAN K. ET AL: "Kinetic Parameters Estimation for Three Way Catalyst Modeling", INDUSTRIAL & ENGINEERING CHEMISTRY RESEARCH, vol. 50, no. 17, 7 September 2011 (2011-09-07), pages 9960-9979, XP055957119, ISSN: 0888-5885, DOI: 10.1021/ie200726j * the whole document * ----- -/-- | 1-15 | |

TECHNICAL FIELDS SEARCHED (IPC)

G16C

The present search report has been drawn up for all claims

| Place of search | Date of completion of the search | Examiner |
|---|---|---|
| The Hague | 15 September 2022 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt
European
Patent Office
Office européen
des brevets

# EUROPEAN SEARCH REPORT

Application Number

EP 22 15 8024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BJELIC A. ET AL: "Hydrogenation and hydrodeoxygenation of aromatic lignin monomers over Cu/C, Ni/C, Pd/C, Pt/C, Rh/C and Ru/C catalysts: Mechanisms, reaction micro-kinetic modelling and quantitative structure-activity relationships", CHEMICAL ENGENEERING JOURNAL, vol. 359, 1 March 2019 (2019-03-01), pages 305-320, XP055882325, AMSTERDAM, NL ISSN: 1385-8947, DOI: 10.1016/j.cej.2018.11.107 * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 15 September 2022 | Godzina, Przemyslaw |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................
& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TOULOUPIDIS V.** Catalytic Olefin Polymerization Process Modeling: Multi-Scale Approach and Modeling Guidelines for Micro-Scale/Kinetic Modeling. *Macromol. React. Eng.,* 2014, vol. 8, 508-527
- **SOARES J.B.P.** The Use of Instantaneous Distributions in Polymerization Reaction Engineering. *Macromol. React. Eng.,* 2014, vol. 8, 235-259
- Polymerization Kinetics and the Effect of Reactor Residence Time on Polymer Microstructure. **SOARES J. ; TOULOUPIDIS V.** Multimodal Polymers with Supported Catalysts: Design and Production. Springer International Publishing, 2019
- **TOULOUPIDIS V. ; RITTENSCHOBER G. ; PAULIK C.** An Integrated PRE Methodology for Capturing the Reaction Performance of Single- and Multi-site Type Catalysts Using Bench-Scale Polymerization Experiments. *Macromol. React. Eng.,* 2020
- **TOULOUPIDIS V. ; WURNITSCH C. ; ALBUNIA A. ; GALGALI G.** Connecting Linear Polymers Molecular Structure to Viscoelastic Properties and Melt Flow Index. *Macromol. Theory Simul.,* 2016, vol. 25, 392-402
- **TOULOUPIDIS V.** BorVisc: Method for predicting polymer rheological properties based on MWD. *Case study for LLDPE and HDPE Borealis grades,* 2013